# EUROPEAN PATENT APPLICATION

(11) **EP 4 831 910 A1**
(43) Date of publication of application: **28.10.2026**
(21) Application number: 25172658.4
(22) Date of filing: 25.04.2025
(51) Int. Cl.: G06T 5/70

(54) **PROJECTION BASED DENOISING TO REDUCE ARTIFACTS IN MEDICAL IMAGING**

(71) Applicant: Koninklijke Philips N.V., 5656 AE Eindhoven (NL)
(72) Inventor: THRAN, Axel, Eindhoven (NL); BONTUS, Claas, Eindhoven (NL); SHECHTER, Gilad, Eindhoven (NL); BRENDEL, Bernhard Johannes, Eindhoven (NL)
(74) Representative: Philips Intellectual Property & Standards

(57) **Abstract**

A projection based denoising to reduce artifacts in medical imaging includes processing projection data by applying a frequency-selective denoising technique, such that a high-frequency component undergoes no or less aggressive denoising compared to a low-frequency component. The denoised projection data is then reconstructed to form image data.

## Description

### FIELD OF THE INVENTION

The present disclosure relates to the field of medical imaging and, in particular, to the processing of projection data.

### BACKGROUND OF THE INVENTION

There is an ongoing interest in the performance of non-invasive imaging of a subject or patient. In modern medicine, images produced using such techniques are important for aiding in the performance of diagnosis and analysis of a condition of the subject or patient. A computed tomography (CT) scan provides one approach for performing imaging of the subject.

In particular, CT scanners are well-established medical imaging devices that use a detector to detect an interaction between X-ray radiation and irradiated material in order to generate projection data of an imaged area. The projection data is then reconstructed using an appropriate technique, such as filtered back-projection (FBP), to produce image data.

Other forms of medical imaging devices may be designed to produce projection data during a scanning procedure, including magnetic resonance imaging (MRI) devices and positron emission tomography (PET) imaging devices.

There is an ongoing desire to reduce the noise present in image data produced using any such medical devices.

In particular, for a CT scanner, noise is more prominent in image data when performing the imaging scan using a relatively low dose of X-ray radiation. Reliable and accurate noise reduction techniques thereby allow for lower dosages of X-ray radiation, i.e., ionizing radiation, to be applied to a subject during a CT scanning procedure.

### SUMMARY OF THE INVENTION

The invention is defined by the claims.

In accordance with a proposed approach, there is provided a computer-implemented method for processing projection data for a medical imaging system. The method comprises: receiving projection data of a subject, wherein the projection data is generated using the medical imaging system; processing the projection data using a projection denoising procedure to produce denoised projection data, wherein the projection denoising procedure is configured to perform more aggressive denoising of a low-frequency component of the projection data than denoising performed on a high-frequency component of the projection data; and reconstructing the denoised projection data to produce image data.

The present disclosure proposes a mechanism for denoising projection data in which a low-frequency component of the projection data is denoised to a greater extent, i.e., more aggressively, than a high-frequency component. By applying more aggressive denoising to low-frequency components, the proposed approach is able to reduce overall image noise without compromising fine structural information critical for accurate diagnosis. In particular, it has been recognized that denoising at high-frequencies may (e.g., unintentionally) sacrifice high frequency structures in the projection data, and thus leads to smearing of object boundaries in the image data. This effect is enhanced with increased aggressiveness of denoising of the high-frequency component.

In this context, low-frequency component of projection data may refer to portions of the projection data that represent larger, more gradual variations or broader structures in the imaged subject. Contrarily, the term high-frequency component of projection data may refer to portions of the projection data that represent finer details, sharp edges, or rapid variations in the imaged subject.

More specifically, the term "low-frequency component of projection data" may refer to portions of the projection data with spatial frequencies below a threshold value. Similarly, the term "high-frequency component of projection data" may refer to portions of the projection data with spatial frequencies above the threshold value. The specific value of the threshold value may depend, for instance, upon the resolution of the medical imaging system and/or user requirements.

In some examples, the projection denoising procedure comprises: splitting the projection data into low-frequency projection data and high-frequency projection data; processing the low-frequency component of the projection data using a first projection denoising subprocedure to produce first denoising data; defining second denoising data using the high-frequency component of the projection data; and combining at least the first denoising data and the second denoising data to produce the denoised projection data.

This approach allows for tailored denoising of different frequency components, improving noise reduction while maintaining image quality. The separate processing of low and high-frequency data enables fine-tuned control over the denoising process, resulting in improved overall image clarity.

In some examples, defining the second denoising data comprises processing the high-frequency component of the projection data using a second projection denoising subprocedure to produce the second denoising data, wherein the second projection denoising subprocedure is different to the first projection denoising subprocedure. This differentiated approach to processing high-frequency data allows for preservation of detail while still reducing noise. By employing a distinct procedure for high-frequency components, the method ensures that fine structures and edges are maintained, enhancing the diagnostic value of the resulting images.

In some examples, the second projection denoising subprocedure is less aggressive than the first projection denoising subprocedure. Using a less aggressive projection denoising subprocedure for high-frequency data helps preserve important fine details in the image data. This approach strikes a balance between noise reduction and retention of critical high-frequency information, resulting in clearer, more diagnostically useful image data.

In some examples, defining the second denoising data comprises defining the high-frequency component of the projection data as the second denoising data. This approach preserves all high-frequency information in the original projection data, ensuring that fine details and sharp edges are maintained in the final image. By retaining the original high-frequency data, the method minimizes or reduces the risk of losing crucial diagnostic information during the denoising process.

In some examples, splitting the projection data comprises: processing the projection data using a low-pass filter to produce the low-frequency component of the projection data; and subtracting the low-frequency component of the projection data from the projection data to produce the high-frequency component of the projection data.

This splitting technique effectively separates low and high-frequency components, allowing for targeted denoising. The use of a low-pass filter ensures a clean separation of frequency components, enabling more precise and effective noise reduction strategies for each component. Use of this splitting mechanism also helps to ensure that all the information contained in the projection data is preserved across the combination of the low-frequency and high-frequency projection data, thereby achieving more faithful and accurate processing of the projection data.

In some examples, splitting the projection data comprises: converting the projection data into a frequency domain to produce frequency-domain projection data; converting a first portion of the frequency domain data that lies below a predetermined frequency to spatial domain to produce the low-frequency component of the projection data; and converting a second portion of the frequency domain data that lies above the predetermined frequency to spatial domain to produce the high-frequency component of the projection data.

This frequency domain-based splitting approach allows for precise separation of low and high-frequency components.

In some examples, the first projection denoising subprocedure comprises: downsampling the low-frequency component of the projection data to produce downsampled low-frequency projection data; denoising the downsampled low-frequency projection data to produce denoised downsampled low-frequency projection data; and upsampling the denoised downsampled low-frequency projection data to produce the first denoising data.

This downsampling-based approach reduces computational complexity while effectively denoising a low-frequency component of the projection data. By operating on downsampled data, this technique allows for faster or more efficient processing of low-frequency components.

In some examples, the projection denoising procedure comprises: processing the projection data to produce residual low-frequency projection data, which represents an estimate of low-frequency noise contained in the projection data; and processing at least the projection data and the residual low-frequency projection data to produce the denoised projection data.

This residual-based approach allows for targeted removal of estimated low-frequency noise. By isolating and addressing the low-frequency noise component, the method is able to achieve more effective overall noise reduction while reducing impact on important image features.

In some examples, processing the projection data to produce residual low-frequency projection data comprises: downsampling the projection data to produce downsampled projection data; denoising the downsampled projection data to produce residual downsampled projection data, which represents an estimate of noise contained in the downsampled projection data; and upsampling the residual downsampled projection data to produce the residual low-frequency projection data.

This approach for estimating low-frequency noise combines the benefits of downsampling with targeted noise estimation. The method allows for efficient processing of large datasets while focusing on the most relevant noise components, resulting in more effective and computationally efficient denoising.

In some examples, the method further comprises processing the image data using an image denoising procedure to produce denoised image data.

This additional denoising step in the image domain functions to further enhance image quality. By applying denoising at multiple stages of the imaging pipeline, the method can address different types of noise more effectively, resulting in cleaner, more diagnostically valuable final images.

In some examples, in the image denoising procedure, the aggressiveness of denoising a high-frequency component of the image data may be no less than the aggressiveness of denoising a low-frequency component thereof.

In the image domain, allowing for equal or more aggressive denoising of high-frequency components can help further reduce noise artifacts that may have persisted through the reconstruction process. This strategy recognizes that some noise characteristics may manifest differently in the image domain compared to the projection domain.

By permitting more aggressive denoising of high-frequency components in the image domain, the method can target residual noise that often appears as fine-grained patterns or speckles in the reconstructed image.

There is also provided a device for processing projection data for a medical imaging system. The device comprises: processing circuitry; and a memory containing instructions that, when executed by the processing circuitry, configure the processing circuitry to: receive projection data of a subject, wherein the projection data is generated using the medical imaging system; process the projection data using a projection denoising procedure to produce denoised projection data, wherein the projection denoising procedure is configured to perform more aggressive denoising of a low-frequency component of the projection data than denoising performed on a high-frequency component of the projection data; and reconstruct the denoised projection data to produce image data.

The projection denoising procedure may comprise: processing the projection data to produce residual low-frequency projection data, which represents an estimate of low-frequency noise contained in the projection data; and processing at least the projection data and the residual low-frequency projection data to produce the denoised projection data There is also provided a system comprising: the device as described above; and the medical imaging system configured to generate the projection data of the subject.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system in which proposed embodiments may be employed;
Fig. 2 is a flowchart illustrating a proposed method in accordance with one embodiment of the present invention;
Fig. 3 is a flowchart illustrating another proposed method in accordance with another embodiment of the present invention;
Fig. 4 is a flowchart illustrating another proposed method in accordance with yet another embodiment of the present invention;
Fig. 5 is a flowchart illustrating another proposed method in accordance with still another embodiment of the present invention;
Fig. 6 is a flowchart illustrating another proposed method in accordance with still another embodiment of the present invention;
Fig. 7 is a flowchart illustrating another proposed method in accordance with still another embodiment of the present invention; and
Fig. 8 illustrates an effect of employing the proposed method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for processing projection data that includes reducing noise in the projection data. The mechanism comprises applying a frequency-selective denoising technique to the projection data, such that a high-frequency component undergoes no or less aggressive denoising compared to a low-frequency component. The denoised projection data is reconstructed to form image data.

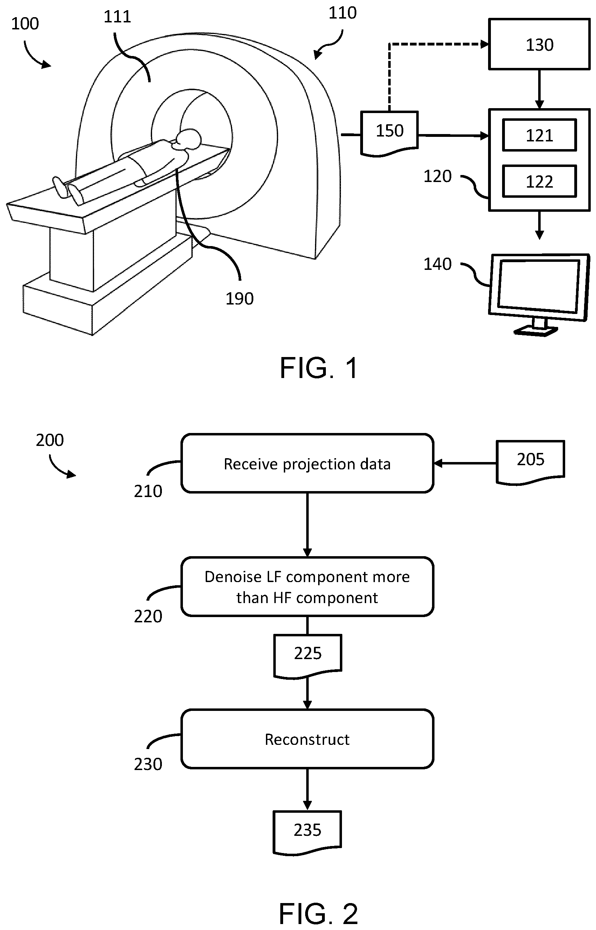

Fig. 1 illustrates a system 100 in which embodiments may be employed for improved contextual understanding. The system 100 comprises a CT scanning system 110 and a device 120.

The CT scanning system 110 is configured to capture and/or generate CT projection data 150 of a subject 190 during a single scanning procedure. In this context, the projection data comprises the raw data captured by the CT scanning system 110 during the single scanning procedure before reconstruction has been performed.

By way of explanation, the projection data may be captured by iteratively sampling a set of projection data elements using a detection system 111, as the subject 190 and detection system 111 are moved with respect to one another. Each set therefore represents data captured during a single, respective capture period, i.e., during one iteration of the sampling. In this way, the projection data comprises a sequence of sets of projection data elements.

In general, during a scanning procedure, the detection system rotates around the subject. The rotation of the detection system with respect to the subject is definable using a rotation angle, also known as a projection angle. In particular, each set of projection data elements may be associated with a respective rotation/projection angle, representing the rotation angle of the detection system with respect to the subject during capture of the projection data elements of the set.

Known types of scanning procedures include circular CT scanning procedures and helical CT scanning procedures. The function and design of CT scanning systems for carrying out such CT scanning procedures are well known and established in the art.

The device 120 comprises processing circuitry 121 and a memory 122. The memory contains instructions that, when executed by the processing circuitry, configure the processing circuitry to perform one or more tasks or functions. The device 120 may, for instance, be replaced by any other form of processing system.

The device 120 may be communicatively coupled to the CT scanning system 110 so as to receive at least projection data 150 from the CT scanning system. The communicative coupling may be wired or wireless, and approaches are known in the art.

In other approaches, the CT scanning system 110 may store the projection data 150 in a data storage 130 (which may form a part of the system 100). The device 120 may be communicatively coupled to the data storage, e.g., to receive or obtain projection from the data storage 130. The communicative coupling may be wired or wireless, and approaches are known in the art.

The processing circuitry 121 may include, but is not limited to, one or more of the following: conventional microprocessors, application specific integrated circuits (ASICs), and/or field-programmable gate arrays (FPGAs). The memory 122 may comprise any volatile and/or non-volatile computer memory such as RAM, PROM, EPROM, and EEPROM. The instructions contained in the memory may effectively define one or more programs that, when executed on the processing circuitry, cause the processing circuitry to perform encoded functions.

As a typical use-case scenario, the processing system 120 is often configured to obtain or receive the projection data 150, produced by the CT scanning system 110, and perform reconstruction on the projection data 150 to thereby produce CT image data. The processing system 120 may then control a user interface 140, e.g., a screen, to provide a visual representation of the CT image data. Approaches for controlling a user interface in this way are well established in the art.

The present disclosure describes an improved mechanism for performing reconstruction of the projection data. In particular, the proposed approach facilitates the generation of image data with reduced noise and with a reduced risk of blurring in the image data.

Although the above described system makes use of a CT scanning system 110 to produce projection data, the skilled person will readily appreciate how any other suitable form of medical imaging device or system could be employed to produce projection data to be processed according to the herein proposed method, mechanism and/or processing circuitry. For instance, the CT scanning system 110 may be replaced with any other suitable medical imaging system capable of producing projection data, such as an MRI system or a PET system. Other suitable medical imaging systems capable of producing projection data will be readily apparent to the skilled person.

Bearing this in mind, for the sake of explanative clarity, the following description will assume that the projection data is produced using a CT scanning system.

Fig. 2 is a flowchart illustrating a proposed computer-implemented method 200 for processing projection data 205 for a medical imaging system.

The method 200 comprises receiving 210 projection data 205 of a subject. The projection data is generated using the medical imaging system, examples of which have been previously described.

The projection data 205 may be received/obtained directly from the medical imaging system itself. Alternatively, the projection data may be received/obtained from a memory or data storage communicatively connected to the medical imaging system. Other approaches for storing, providing and receiving projection data will be apparent to the skilled person.

In some examples, receiving 210 projection data 205 of the subject further comprises preprocessing the projection data. There is a wide variety of well-known approaches for preprocessing projection data, e.g., to normalize the projection data, linearize the projection data, resample and/or rebin projection data, and so on.

The method 200 also comprises processing the projection data using a projection denoising procedure 220 to produce denoised projection data 225. In other words, the method 200 comprises performing a projection denoising procedure on the projection data.

The projection denoising procedure 220 is configured to perform more aggressive denoising of a low-frequency component of the projection data than denoising performed on a high-frequency component of the projection data.

The projection denoising procedure 220 may be performed by separating the low-frequency component from the high-frequency component, and performing more aggressive denoising of the separated low-frequency component than any denoising performed on the high-frequency component. In some variants, no denoising of the high-frequency component is performed. In other variants, some denoising of the high-frequency component is performed, but less aggressively than on the low-frequency component.

Alternatively, the projection denoising procedure 220 may be performed by performing a technique that performs denoising of only the low-frequency component of the projection data.

More detailed examples for approaches for performing the projection denoising procedure in the projection denoising procedure 220 are described later.

In the context of the present disclosure, the projection data is treated analogously to image data, such that low-frequency content of projection data comprises large features of the projection data, and high-frequency content of projection data comprises smaller, more granular features of the projection data.

The method 200 also comprises reconstructing the denoised projection data 225 to produce image data 235.

A suitable approach to performing the act of reconstructing is to perform back projection, such as filtered back-projection. Other suitable examples include the algebraic reconstruction technique (ART), the simultaneous algebraic reconstruction technique (SART), and ordered subset expectation maximization (OSEM).

Appropriate approaches for performing reconstruction of filtered and denoised projection data are widely known in the art, including those that are based upon the inverse of the Radon transform (i.e., back projection), as explained by, inter alia, Beckmann, Matthias, and Armin Iske "Error estimates and convergence rates for filtered back projection" Mathematics of Computation 88.316 (2019): 801-835. Another approach is suggested by United States Patent Application No. US 2024/185485 A1, filed 12 April 2022.

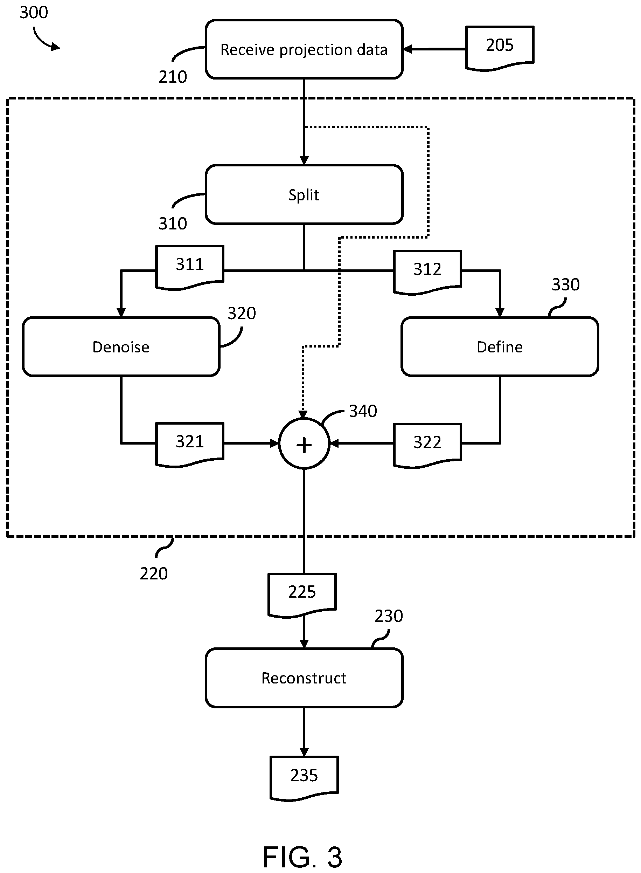

Fig. 3 is a flowchart illustrating a proposed computer-implemented method 300 for processing projection data 205 for a medical imaging system. The method comprises performing the acts 210, 220, 230 as previously disclosed.

In particular, Fig. 3 illustrates an example approach for performing the projection denoising procedure 220.

In this approach, the projection denoising procedure 220 comprises splitting 310 the projection data 205 into the low-frequency component 311 of the projection data (low-frequency projection data) and the high-frequency component 312 of the projection data (high-frequency projection data). Thus, splitting 310 effectively divides the projection data 205 into a low-frequency component, i.e., low-frequency projection data and a high-frequency component, i.e., high-frequency projection data.

In one approach, splitting 310 comprises processing the projection data using a low-pass filter to produce the low-frequency component 311 of the projection data, and subtracting the low-frequency component of the projection data from the projection data to produce the high-frequency component 312 of the projection data. This helps ensure that all the information contained in the projection data is preserved across the combination of the low-frequency and high-frequency components of the projection data, thereby achieving more faithful and accurate processing of the projection data.

For the purposes of this approach, the projection data may be treated analogously to image data. In particular, a spatial low-pass filter may be used, sometimes known as a blurring or a smoothing filter.

When applied to projection data, a spatial low-pass filter may operate on the spatial domain of the projections, similar to how 2D image data is processed. More particularly, splitting the projection data may comprise processing the projection data with a filter kernel, e.g., that convolves with the projection data, to produce the low-frequency component of the projection data. As previously mentioned, the high-frequency component of the projection data may then be obtained by subtracting the low-frequency component of the projection data from the original projection data.

Suitable types of spatial low-pass filters that may be used include Gaussian filters, which apply a weighted average based on a Gaussian distribution, or box/boxcar filters, also called moving average filters, which perform a simple averaging over a defined neighborhood, or sinc filters, which apply a sinc function to the spatial domain to attenuate high frequencies. The choice of filter and its parameters, e.g., kernel size, can be tuned to achieve the desired frequency separation.

In another approach, splitting 310 the projection data comprises downsampling or fusing the projection data to produce downsampled projection data. The downsampled projection data effectively represents a low-frequency content of the projection data, albeit, at a lower resolution. Splitting the projection data may further comprise producing the low-frequency component of the projection data by upsampling the downsampled projection data, i.e., to the original resolution of the projection data, and producing the high-frequency component of the projection data by subtracting the low-frequency component of the projection data from the original projection data.

In another approach, splitting 310 the projection data comprises converting the projection data into a frequency domain to produce frequency-domain projection data, converting a first portion of the frequency domain data that lies below a predetermined frequency to spatial domain to produce the low-frequency component of the projection data, and converting a second portion of the frequency domain data that lies above the predetermined frequency to spatial domain to produce the high-frequency component of the projection data. This provides a more computationally efficient mechanism for splitting projection data into low-frequency and high-frequency components.

Similarly, for the purposes of this approach, the projection data may be treated analogously to image data. Thus, converting the projection data into the frequency domain may be performed using a spatial Fourier-based transform. **In** the context of 2D/3D projection data processing, the 2D Discrete Fourier Transform (DFT) or its fast implementation, the 2D Fast Fourier Transform (FFT), may be used to perform this procedure in act 310.

In some examples, for the purposes of splitting 310 the projection data, the low-frequency data may contain a majority of the content, e.g., more than 90% of the content, of the projection data for frequencies lower than a predetermined number of line pair per cm. A line pair per cm is a measure of spatial resolution commonly used in medical imaging. In particular, this measure represents the number of alternating light and dark lines that can be resolved or distinguished from one another within one centimeter of image space.

A higher number of line pairs per cm corresponds to a higher spatial frequency, while a lower number of line pairs per cm represents a lower spatial frequency.

Appropriate selection of blurring or smoothing filters to process the projection data will effectively reduce the number of line pairs per cm within the projection data. Accordingly, by applying these filters, the spatial resolution of the projection data is deliberately decreased to effectively isolate the lower frequency information. The parameters and type of filter used, such as kernel size and shape, may be selected to achieve the desired frequency separation. A larger kernel size may result in more aggressive smoothing, e.g., for a same type of smoothing filter, further reducing the number of line pairs per cm.

The value of the predetermined number of line pairs per centimeter functions as a cutoff point for separating low and high-frequency components. In some examples, the predetermined number may, for instance, take a value of 12±5. **In** other words, the effective resolution of the projection image may be filtered or reduced to 12±5 line pairs per cm to define the low-frequency component of the projection data. The proposed range, 7 to 17 line pairs per cm, is particularly advantageous in the context of projection data for medical imaging, as it corresponds to the transition between larger anatomical structures and finer details.

The projection denoising procedure 220 further comprises processing 320 the low-frequency component 311 of the projection data using a first projection denoising subprocedure to produce first denoising data 321.

The first denoising data 321 may be a denoised version of the low-frequency component 311 of the projection data, i.e., denoised low-frequency projection data, or first residual projection data representing the predicted or determined noise content of the low-frequency component of the projection data.

Any suitable projection denoising mechanism may be employed for the purposes of the first projection denoising subprocedure. Any such projection denoising procedure or subprocedure may be labelled a projection domain denoising subprocedure.

By way of example only, one suitable projection denoising procedure makes use of one or more suitably trained machine-learning algorithms to process the low-frequency component of the projection data to produce the first denoising data. Suitable examples of machine-learning algorithms are later described.

Examples of projection domain projection denoising procedures are provided or identified in Sun, Jingzhang, et al. "Deep learning-based denoising in projection-domain and reconstruction-domain for low-dose myocardial perfusion SPECT" Journal of Nuclear Cardiology 30.3 (2023): 970-985; Zhang, Hao, et al. "Applications of nonlocal means algorithm in low-dose X-ray CT image processing and reconstruction: a review" Medical physics 44.3 (2017): 1168-1185; and Zhang, Haiyan, et al. "Projection domain denoising method based on dictionary learning for low-dose CT image reconstruction" Journal of X-ray Science and Technology 23.5 (2015): 567-578.

United States Patent Application having publication No US 2024/095885 A1 discloses an approach for generating a residual noise image by processing image data. The same procedure may be applied to process the low-frequency component of the projection data to produce the first denoising data, such that the first denoising data is effectively residual noise projection data.

The projection denoising procedure 220 comprises defining 330 second denoising data 322 using the high-frequency component 312 of the projection data.

In a simple approach, defining 330 second projection data comprises defining the high-frequency component of the projection data as the second denoising data 322. In other words, the high-frequency component of the projection data can be directly treated as the second denoising data. This avoids a need to perform additional processing on the high-frequency component of the projection data.

In one variant, defining 330 second projection data may comprise processing the high-frequency component of the projection data using an identity function to produce the second denoising data. In this way, the second high-frequence projection data is effectively a copy of the high-frequency component of the projection data.

In another variant, defining 330 second projection data may comprise processing the high-frequency component of the projection data using a second projection denoising subprocedure to produce the second denoising data. In this way, the second denoising data may be denoised high-frequency projection data. The second projection denoising subprocedure may be different than the first projection denoising subprocedure, e.g., employ one or more different parameters. Suitable examples of projection denoising subprocedures have been previously disclosed.

In some variants, the second projection denoising subprocedure is less aggressive than the first projection denoising subprocedure. This can be achieved through, for instance, appropriate selection of parameters or values of each projection denoising subprocedure.

For the sake of completeness, it is noted that the second denoising data may comprise or be second residual projection data representing the predicted or determined noise content of the high-frequency component of the projection data. Approaches for processing a component of projection data to produce residual projection data have been previously described.

The projection denoising procedure 220 also comprises combining 340 the first denoising data and the second denoising data to produce the denoised projection data 225.

In a simple example, combining the first denoising data and the second denoising data comprises adding or summing the first denoising data 321 and the second denoising data 322 to produce the denoised projection data 225.

In another example, combining the first denoising data and the second denoising data comprises performing a weighted addition or summing of the first denoising data and the second denoising data to produce the denoised projection data. The weighted addition or summing makes use of one or more weights, e.g., different weights applied to the first denoising data and the second denoising data. The value of the weights may be defined or adjusted responsive to, for instance, noise levels, desired image characteristics and/or one or more other characteristics.

These two approaches, and similar alternatives, are possible when the first denoising data 321 and the second denoising data 322 each represent a denoised version of the low-frequency component of the projection data and the high-frequency component of the projection data, respectively.

In other scenarios, the first denoising data 321 and the second denoising data 232 each comprise residual noise data for the respective low-frequency component of the projection data or high-frequency component of the projection data.

In such scenarios, combining 340 the first denoising data and the second denoising data should comprise subtracting the first denoising data and the second denoising data from the original projection data to produce the denoised projection data 225.

In yet other scenarios, the first denoising data 321 comprises residual noise data for the low-frequency component of the projection data, and there is no desire to perform denoising of a high-frequency component. In this approach, act 330 can be omitted, and combining the first denoising data and the second denoising data 340 may simply comprise subtracting the first denoising data 321 from the original projection data 205.

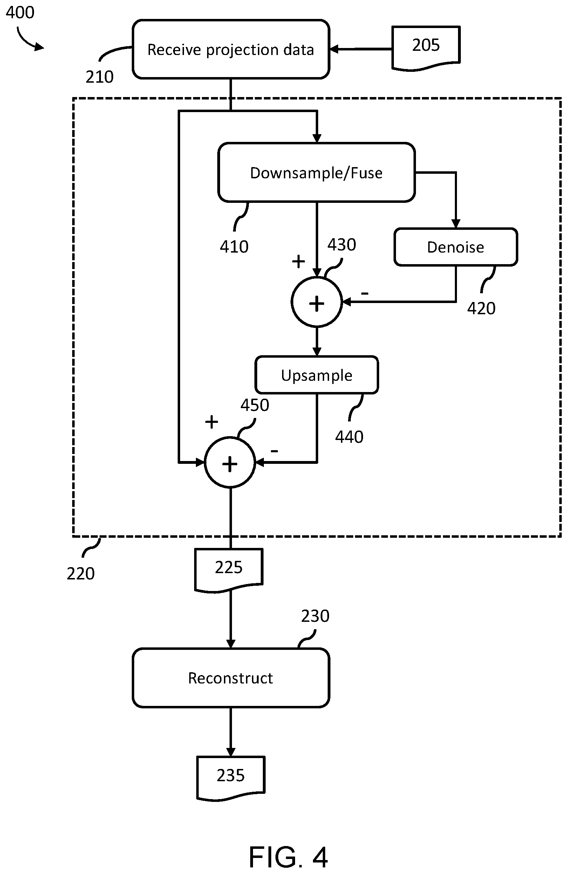

Fig. 4 is a flowchart illustrating a proposed computer-implemented method 400 for processing projection data 205 for a medical imaging system. The method comprises performing the acts 210, 220, 230 as previously disclosed.

In particular, Fig. 4 illustrates another example approach for performing the projection denoising procedure 220.

In this approach, the projection denoising procedure 220 comprises downsampling or fusing 410 the projection data to produce downsampled projection data, i.e., at a lower resolution than the resolution of the projection data 205. The downsampled projection data effectively represents a low-frequency component of the projection data, albeit - at a lower resolution.

In some examples, the downsampling or fusing 410 may comprise processing the projection data using a pooling layer to effectively produce a downsampled version of the projection data, i.e., the downsampled projection data. Suitable pooling layers are well known and employed in neural network technology, and may be employed for a similar purpose here.

In practice, the downsampling/fusing 410 may comprise dividing the projection data into discrete regions of a fixed size, known as pooling windows. Each region comprises an array or matrix of two or more data values of the projection data. Each region is then processed using a particular function to define a respective single value for the sampled projection data from the values contained in said region. The particular function may be a maximum function which selects the largest data value in the region, a minimum function which selects the lowest data value in the region, an average function which averages all data values in the region, or any other function that processes two or more values to produce a single output value representative of the data represented by the two or more values.

The projection denoising procedure 220 here further comprises denoising 420 the downsampled projection data. In this approach, act 420 comprises producing a denoised version of the downsampled projection data, i.e., denoised downsampled projection data. This effectively represents denoised low-frequency image data, albeit at a lower resolution than the original projection data 205. Any herein disclosed approach for denoising projection data may be employed to a similar effect for the purposes of act 420.

The projection denoising procedure 220 also comprises subtracting 430 the denoised downsampled projection data from the downsampled projection data. This produces residual downsampled projection data, which represents an estimate of noise contained in the downsampled projection data produced by act 410.

The projection denoising procedure 220 also comprises upsampling 440 the residual downsampled projection data, e.g., to the resolution of the original projection data 205. This effectively produces residual low-frequency projection data, which represents an estimate of low-frequency noise contained in the projection data, i.e., the noise in the low-frequency component of the projection data 205.

The projection denoising procedure 220 here also comprises subtracting 450 the residual low-frequency projection data, i.e., the output of act 440, from the original projection data 205. This produces the denoised projection data 225 for further processing.

As an alternative to performing acts 420 and 430, the downsampled projection data may be processed with a different form of technique that, e.g., directly, produces the residual downsampled projection data, i.e., representing the noise predicted or estimated to be contained in the downsampled projection data. This avoids the need to perform the subsequent subtracting act 430.

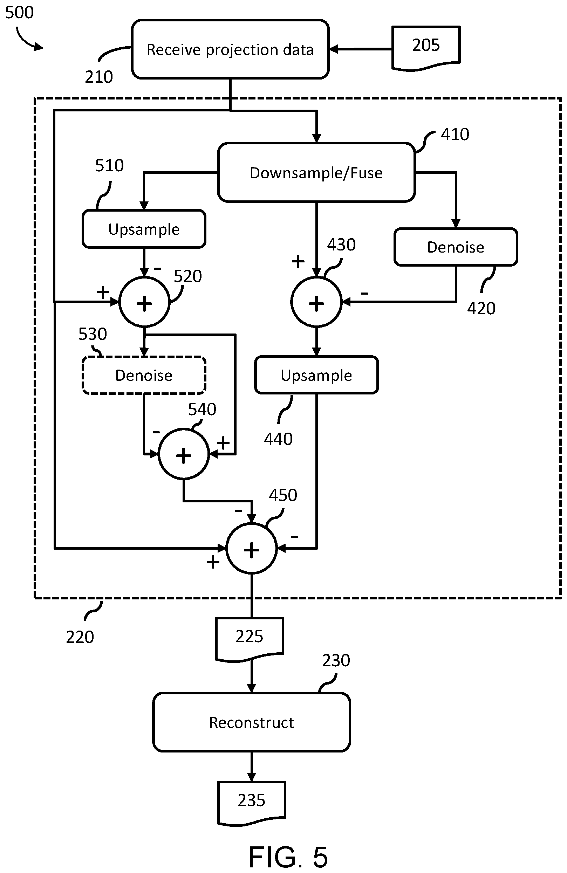

Fig. 5 is a flowchart illustrating a proposed computer-implemented method 500 for processing projection data 205 for a medical imaging system. The method comprises performing the acts 210, 220, 230 as previously disclosed.

The method 500 differs from the previously disclosed method 400 by comprising additional steps/acts and in that the subtracting 450 is modified. Other acts/steps may be embodied as previously disclosed.

In method 500, the projection denoising procedure 220 further comprises an act 510 of upsampling the downsampled projection data, e.g., to the resolution of the original projection data 205, to produce resampled projection data. Act 510 may be performed in a similar/analogous manner to the previously disclosed act of upsampling 440 (Fig. 4). This resampled projection data effectively represents a low-frequency component of the projection data in the same resolution as the original projection data 205.

The projection denoising procedure 220 here further comprises subtracting 520 the re-upsampled projection data from the original projection data, to produce modified projection data. This modified projection data effectively represents a high-frequency component of the projection data in the same resolution as the original projection data 205.

The projection denoising procedure 220 here further comprises denoising 530 the modified projection data using a projection denoising procedure to produce denoised modified projection data. This effectively represents a denoised version of the high-frequency component of the original projection data. Any herein disclosed approach for denoising projection data may be employed to a similar effect for the purposes of act 420.

The denoising performed in act 530 is less aggressive than performed in act 420.

The projection denoising procedure 220 here further comprises subtracting 540 the denoised modified projection data from the modified projection data to produce residual high-frequency projection data, representing the noise predicted or estimated to be contained in the modified projection data, i.e., in the high-frequency component of the projection data.

As an alternative to performing acts 530 and 540, the modified projection data may be processed with a different form of denoising technique that, e.g., directly, to produce the residual high-frequency projection data, i.e., modified projection data. This avoids the need to perform the subtracting act 540.

The subtracting 450 is modified to instead comprise subtracting the residual low-frequency projection data and the residual high-frequency projection data from the original projection data 205. This produces the denoised projection data 225.

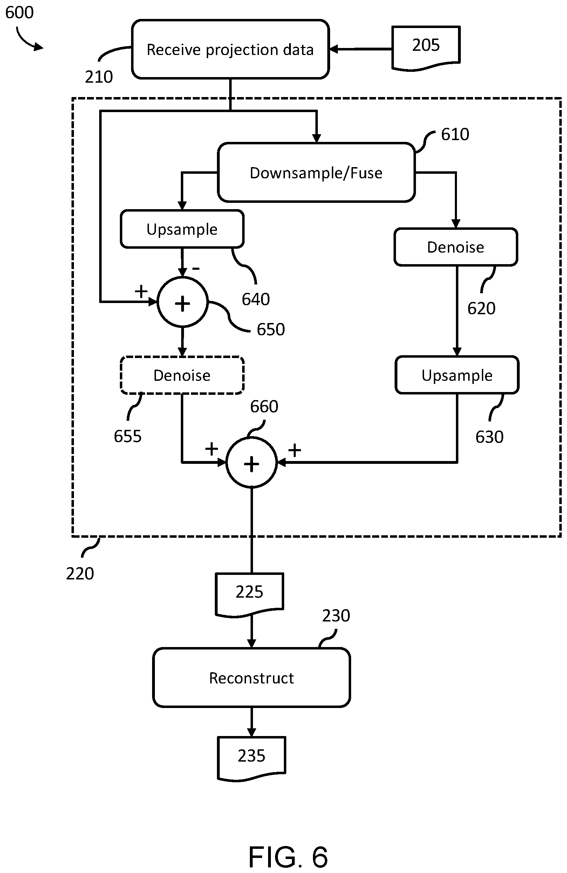

Fig. 6 is a flowchart illustrating a proposed computer-implemented method 600 for processing projection data 205 for a medical imaging system. The method comprises performing the acts 210, 220, 230 as previously disclosed.

For the method 500, the projection denoising procedure 220 of processing the projection data comprises downsampling or fusing 410 the projection data to produce downsampled projection data. This may be performed using any previously disclosed approach, e.g., with reference to act 410 of method 400 (Fig. 4).

The projection denoising procedure 220 here also comprises denoising 620 the downsampled projection data, to produce denoised downsampled projection data. In this approach, act 620 comprises producing a denoised version of the downsampled projection data, i.e., denoised downsampled projection data. This effectively represents denoised low-frequency projection data, albeit at a lower resolution than the original projection data 205. Any herein disclosed approach for denoising projection data may be employed to a similar effect for the purposes of act 420.

The projection denoising procedure 220 here also comprises upsampling 630 the denoised downsampled projection data, e.g., to the resolution of the original projection data 205. This produces denoised low-frequency projection data.

The projection denoising procedure 220 here also comprises upsampling 640 the downsampled projection data, e.g., to the resolution of the original projection data 205, to produce resampled projection data. Act 640 may be performed in a similar/analogous manner to the previously disclosed act of upsampling 510 (Fig. 5).

The projection denoising procedure 220 here further comprises subtracting 650 the re-upsampled projection data from the original projection data, to produce modified projection data. This modified projection data effectively represents a high-frequency component of the projection data in the same resolution as the original projection data 205.

In some embodiments, the projection denoising procedure 220 may comprise denoising 655 the modified projection data, to produce denoised modified projection data, i.e., denoised high-frequency projection data. If performed, any herein disclosed approach for denoising projection data may be employed to a similar effect for the purposes of act 655.

The projection denoising procedure 220 further comprises combining 660 the denoised low-frequency projection data and either the modified projection data or, if produced, the denoised high-frequency projection data. This produces the denoised projection data.

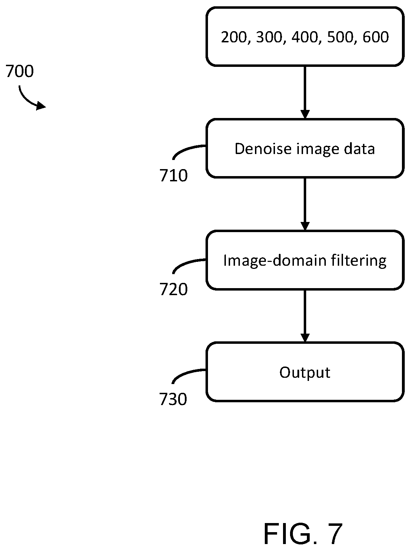

Fig. 7 is a flowchart illustrating a variant method 700 that performs additional optional acts to the image data. In particular, the method 700 comprises performing the acts of any previously disclosed method 200, 300, 400, 500, 600, as well as one or more optional acts or steps.

The method 700 may comprise an act 710 of processing the image data using an image denoising algorithm to produce denoised image data. In other words, act 710 may comprise denoising the image data, i.e., in the image domain.

Image denoising algorithms for use in processing image data derived from projection data are well known in the art.

Examples include those set out by Thanh, Dang, and Prasath Surya "A review on CT and X-ray images denoising methods" Informatica 43.2 (2019); and/or Yang, Qingsong, et al. "Low-dose CT image denoising using a generative adversarial network with Wasserstein distance and perceptual loss" IEEE transactions on medical imaging 37.6 (2018): 1348-1357.

Other approaches for performing denoising of image data derived from projection data are known in the art, e.g., as discussed by Mohan, J., V. Krishnaveni, and Yanhui Guo "A survey on the magnetic resonance image denoising methods" Biomedical signal processing and control 9 (2014): 56-69

In some examples, in the image denoising algorithm, the aggressiveness of denoising a high-frequency component of the image data may be no less than the aggressiveness of denoising a low-frequency component thereof.

The method 700 may comprise an act 720 of processing the image data, or denoised image data if act 710 is performed, using image-domain filtering to produce output image data, wherein image-domain filtering is configured to performing filtering on the denoised image data in accordance with the filter characteristics (e.g. MTF, NPS) of one or more predefined scan protocols.

In other words, act 720 may comprise filtering the image data in an image-domain to produce the output image data.

This approach recognizes that there may be a preference or desire for image data to have a particular appearance and/or one or more characteristic(s). Using such image-domain filtering thereby allows the image data to be adapted for any such preferences, e.g., to achieve a desired contrast distribution and so on.

The method 700 may further comprise an act 730 of outputting the image data and/or any further data derived from the image data, such as the denoised and/or output image data previously described. Act 730 may comprise outputting the image data to another device, e.g., separate to the device that carried out and/or performed the method 200.

For instance, act 730 may comprise controlling a user interface to provide a visual representation of the image data or, if produced, the output image data. Step 730 may thereby effectively comprise displaying the image data or, if produced, the output image data.

In some examples, act 730 comprises storing the image data or, if produced, the output image data. The image data may be stored in a memory and/or data storage.

In some examples, act 730 comprises passing the image data and/or any further data derived therefrom to another device for further processing and/or analysis.

Any herein proposed computer-implemented method may, for instance, be performed by the device 120 previously described (Fig. 1). More particularly, the computer-implemented method may be performed by the processing circuitry 121 of the device 120 when executing instructions contained in the memory 122.

It has previously been explained how example projection denoising procedures may make use of one or more machine-learning algorithms to perform denoising.

A machine-learning algorithm is any self-training/self-learning algorithm that processes input data in order to produce or predict output data. Here, the input data comprises projection data and the output data comprises corresponding denoising data, e.g., a denoised version of the portion of projection data or residual noise of the portion of projection data.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (neural network) is inspired by the human brain. Neural networks are comprised of layers, each layer comprising a plurality of neurons. Each neuron comprises a mathematical operation. In particular, each neuron may comprise a different weighted combination of a single type of transformation, e.g. the same type of transformation, sigmoid etc. but with different weightings. In the process of processing input data, the mathematical operation of each neuron is performed on the input data to produce a numerical output, and the outputs of each layer in the neural network are fed into the next layer sequentially. The final layer provides the output.

A decision tree algorithm processes input data through a tree of nodes. In the tree of nodes, each successive node splits into two or more further nodes until reaching a terminal or end node. When performing the decision tree algorithm using the tree of nodes, at each node, a decision is made as to which further node to move to next based on the input data. The end node defines the outcome of the decision tree algorithm, and therefore the machine-learning algorithm.

Methods of training a machine-learning algorithm are well known. Typically, such methods comprise obtaining a training dataset, comprising training input data entries and corresponding training output data entries. The training input data entries provide example inputs for the machine-learning algorithm and the training output data entries provide target outputs for the machine-learning algorithm when processing the corresponding example inputs.

For many machine-learning algorithms, such as a neural network or decision tree, training is performed by applying an initialized machine-learning algorithm to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, weightings of the mathematical operation of each neuron may be modified until the error converges. Known methods of modifying a neural network include gradient descent, backpropagation algorithms and so on.

Other approaches for training machine-learning algorithms, e.g., decision trees, are known in the art. For instance, decision trees are often trained using a decision tree builder or learning techniques, such as those set out by Suthaharan, Shan, and Shan Suthaharan "Decision tree learning" Machine Learning Models and Algorithms for Big Data Classification: Thinking with Examples for Effective Learning (2016): 237-269 or Ruggieri, Salvatore "Yadt: Yet another decision tree builder" 16th IEEE International Conference on Tools with Artificial Intelligence. IEEE, 2004.

For the purposes of performing act 230, the training input data entries correspond to example instances of low-frequency projection data or high-frequency projection data. The training output data entries may correspond to instances of denoised low-frequency/high-frequency projection data.

One approach to generating the training input data entries and the training output data entries can be as follows.

First, collect a plurality of instances of low-frequency/high-frequency projection data, depending upon the intended purpose of the machine-learning algorithm. One approach for collecting such data is to collect a plurality of instances of projection data and split the projection data into low-frequency and high-frequency components using the techniques described earlier. This produces a plurality of instances of low-frequency or high-frequency projection data.

Then, add noise, e.g., pseudo-randomly generated noise, to each of the plurality of instances of low-frequency/high-frequency projection data. This produces two sets of projection data, the first set including the originally collected instances of low-frequency/high-frequency projection data and the second set including the noise-added instances of low-frequency/high-frequency projection data. The first set of instances of low-frequency/high-frequency projection data act as the training output data entries. The second set of filtered instances of low-frequency/high-frequency projection data act as the training input data entries.

Another approach to generating the training input data entries and the training output data entries is hereafter described.

This approach is designed for use where the projection data is produced using a CT scanner, i.e., is CT projection data. **In** this approach, a plurality of pairs of instances of low-frequency/high-frequency projection data are obtained. Each pair of instances of low-frequency/high-frequency projection data comprises a low-frequency/high-frequency component of a first instance of projection data, obtained by a CT scanner using a first dosage of radiation, and a low-frequency/high-frequency component of a second instance of projection data, obtained by the CT scanner using a second dosage of radiation, the second dosage being greater than the first dosage. The first and the second instance of projection data represent a same imaged region. Each instance of low-frequency/high-frequency projection data may then be split into low-frequency and high-frequency components, e.g., using previously described techniques. Each low-frequency/high-frequency component of the first instance of projection data acts as a respective training input data entry. Each low-frequency/high-frequency component of the second instance of projection data acts as a respective training output data entry.

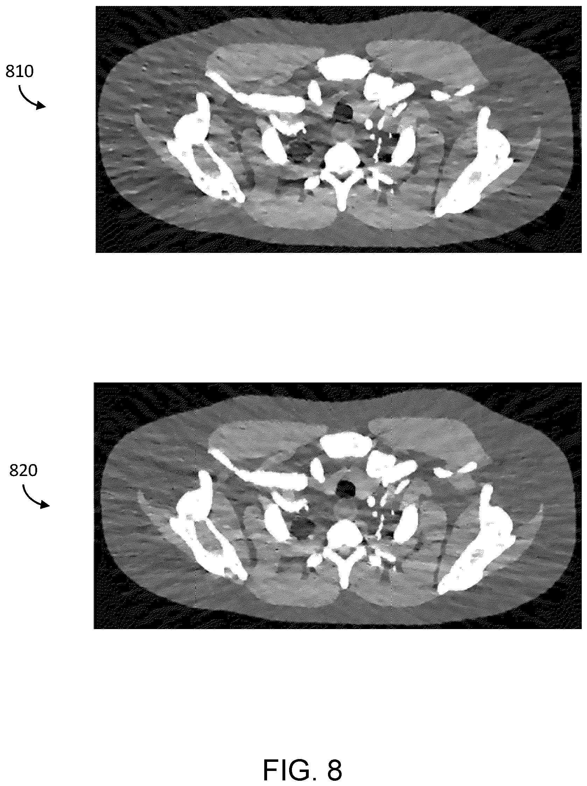

Fig. 8 illustrates the effect of employing the proposed computer-implemented method 200 to process projection data to produce image data.

In particular, Fig. 8 illustrates two separate images, being slices of respective image data, each representing a same anatomical region. A first image 810 is produced using an existing approach, in which all frequency components of the projection data are denoised using a shared denoising technique. A second image 820 is produced using the proposed approach, in which a low-frequency component and a high-frequency component of the projection data are treated differently during denoising, namely, the high-frequency component is less aggressively denoised, e.g., not denoised at all. All other parts of the projection data processing chain are otherwise kept identical, e.g., including a same image domain denoising procedure.

As clearly demonstrated, noise streaks, especially in the outer part of the imaged subject, are substantially reduced with the proposed denoising approach, while the sharpness of object boundaries is not sacrificed. Thus, more clinically useful image data is produced using the proposed process.

The skilled person would be readily capable of developing processing circuitry for carrying out any herein described method, e.g., when executing instructions contained or carried out by a memory. Thus, each step/act of the flow chart may represent a different action performed by processing circuitry, and may be performed by a respective module of the processing circuitry.

Embodiments may therefore make use of processing circuitry. Processing circuitry can be implemented in numerous ways, with software and/or hardware, to perform the various functions required.

A processor is one example of processing circuitry which employs one or more microprocessors that may be programmed using software, e.g., microcode, to perform the required functions. Processing circuitry may however be implemented with or without employing a processor, and also may be implemented as a combination of dedicated hardware to perform some functions and a processor, e.g., one or more programmed microprocessors and associated circuitry, to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, the processing circuitry may be associated with memory, i.e., one or more storage media, such as volatile and non-volatile computer memory such as RAM, PROM, EPROM, and EEPROM. The memory may be encoded with instructions, i.e., one or more programs, that, when executed by the processing circuitry, perform the required functions. Various storage media or medium may be fixed within a device comprising the processing circuitry or may be transportable, such that the one or more programs stored thereon can be loaded into processing circuitry.

Accordingly, there is provided a device for processing projection data for a medical imaging system. The device comprises processing circuitry; and a memory containing instructions that, when executed by the processing circuitry, configure the processing circuitry to perform any herein proposed method.

There is further proposed a system comprising the device and the medical imaging system configured to generate the projection data and/or, if relevant, the initial projection data.

There is also proposed a user interface system comprising the device and a user interface. The device may be configured to control the user interface to provide a visual representation of the image data and/or any further data/information derived from the image data, such as the denoised image data and/or output image data previously described.

The system described above, comprising the device and the medical imaging system, may comprise the user interface system, i.e., further comprise the user interface.

It will be understood that disclosed methods are preferably computer-implemented methods. As such, there is also proposed the concept of a computer program comprising code means for implementing any described method when said program is run on processing circuitry, such as a computer. Thus, different portions, lines or blocks of code of a computer program according to an embodiment may be executed by processing circuitry or computer to perform any herein described method.

There is also proposed a non-transitory storage medium or memory that stores or carries instructions, e.g., a computer program or computer code, that, when executed by processing circuitry, causes the processing circuitry to carry out any herein described method.

In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A computer-implemented method for processing projection data for a medical imaging system, the computer-implemented method comprising:
receiving projection data of a subject, wherein the projection data is generated using the medical imaging system;
processing the projection data using a projection denoising procedure to produce denoised projection data, wherein the projection denoising procedure is configured to perform more aggressive denoising of a low-frequency component of the projection data than denoising performed on a high-frequency component of the projection data; and
reconstructing the denoised projection data to produce image data.

2. The computer-implemented method of claim 1, wherein the projection denoising procedure comprises:
splitting the projection data into the low-frequency component of the projection data and the high-frequency component of the projection data;
processing the low-frequency component of the projection data using a first projection denoising subprocedure to produce first denoising data;
defining second denoising data using the high-frequency component of the projection data; and
combining at least the first denoising data and the second denoising data to produce the denoised projection data.

3. The computer-implemented method of claim 2, wherein defining the second denoising data comprises processing the high-frequency component of the projection data using a second projection denoising subprocedure to produce the second denoising data, wherein the second projection denoising subprocedure is different to the first projection denoising subprocedure.

4. The computer-implemented method of claim 3, wherein the second projection denoising subprocedure is less aggressive than the first projection denoising subprocedure.

5. The computer-implemented method of claim 2, wherein defining the second denoising data comprises defining the high-frequency component of the projection data as the second denoising data.

6. The computer-implemented method of any one of claims 2 to 5, wherein splitting the projection data comprises:
processing the projection data using a low-pass filter to produce the low-frequency component of the projection data; and
subtracting the low-frequency component of the projection data from the projection data to produce the high-frequency component of the projection data.

7. The computer-implemented method of any of claims 2 to 5, wherein splitting the projection data comprises:
converting the projection data into a frequency domain to produce frequency-domain projection data;
converting a first portion of the frequency domain data that lies below a predetermined frequency to spatial domain to produce the low-frequency component of the projection data; and
converting a second portion of the frequency domain data that lies above the predetermined frequency to spatial domain to produce the high-frequency component of the projection data.

8. The computer-implemented method of any one of claims 1 to 7, wherein the first projection denoising subprocedure comprises:
downsampling the low-frequency component of the projection data to produce downsampled low-frequency projection data;
denoising the downsampled low-frequency projection data to produce denoised downsampled low-frequency projection data; and
upsampling the denoised downsampled low-frequency projection data to produce the first denoising data.

9. The computer-implemented method of claim 1, wherein the projection denoising procedure comprises:
processing the projection data to produce residual low-frequency projection data, which represents an estimate of low-frequency noise contained in the projection data; and
processing at least the projection data and the residual low-frequency projection data to produce the denoised projection data.

10. The computer-implemented method of claim 9, wherein processing the projection data to produce the residual low-frequency projection data comprises:
downsampling the projection data to produce downsampled projection data;
denoising the downsampled projection data to produce residual downsampled projection data, which represents an estimate of noise contained in the downsampled projection data; and
upsampling the residual downsampled projection data to produce the residual low-frequency projection data.

11. The computer-implemented method of any one of claims 1 to 10, further comprising denoising the image data to produce denoised image data.

12. A computer program product comprising computer program code means which, when executed on a device having processing circuitry, cause the processing circuitry to perform all of the steps of the method according to any one of claims 1 to 11.

13. A device for processing projection data for a medical imaging system, the device comprising:
processing circuitry; and
a memory containing instructions that, when executed by the processing circuitry, configure the processing circuitry to:
receive projection data of a subject, wherein the projection data is generated using the medical imaging system;
process the projection data using a projection denoising procedure to produce denoised projection data, wherein the projection denoising procedure is configured to perform more aggressive denoising of a low-frequency component of the projection data than denoising performed on a high-frequency component of the projection data; and
reconstruct the denoised projection data to produce image data.

14. The device of claim 13, wherein the projection denoising procedure comprises:
processing the projection data to produce residual low-frequency projection data, which represents an estimate of low-frequency noise contained in the projection data; and
processing at least the projection data and the residual low-frequency projection data to produce the denoised projection data.

15. A system comprising:
the device of claim 13 or 14; and
the medical imaging system configured to generate the projection data of the subject.
